# EUROPEAN PATENT APPLICATION

(11) **EP 4 671 295 A1**
(43) Date of publication of application: **31.12.2025**
(21) Application number: 24780411.5
(22) Date of filing: 27.03.2024
(51) Int. Cl.: C08G 18/70, C08G 18/32, C08G 18/38, C08G 18/73, C08G 18/75, C08G 18/76, G02B 1/04, G02C 7/00

(54) **ISOCYANATE COMPOSITION, POLYMERIZABLE COMPOSITION, RESIN, OPTICAL MATERIAL, AND SPECTACLE LENS**

(30) Priority: 31.03.2023 JP 2023058215
(71) Applicant: Hoya Lens Thailand Ltd., Pathumthani 12130 (TH)
(72) Inventor: KOUSAKA, Masahisa, Tokyo 160-8347 (JP); OHNISHI, Tomofumi, Tokyo 160-8347 (JP); TANIZAWA, Hideya, Tokyo 160-8347 (JP)
(74) Representative: Kraus & Lederer PartGmbB
(86) International application number: PCT/JP2024/012168
(87) International publication number: WO 2024/204305

(57) **Abstract**

There are provided an isocyanate composition including a compound represented by Formula (1) and an isocyanate component, a polymerizable composition including the isocyanate composition, a resin which is a cured product of the polymerizable composition, an optical material including the resin, and a spectacle lens including the resin

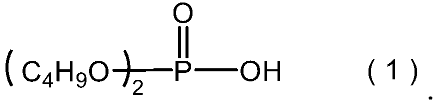

## Description

### [Technical Field]

The present disclosure relates to an isocyanate composition, a polymerizable composition, a resin, an optical material, and a spectacle lens.

### [Background Art]

Plastic spectacle lenses are widely known in the related art. A polythiourethane resin is used as a material for spectacle lenses (for example, PTL 1). Since the polythiourethane resin is a material having an appropriate refractive index, it allows a spectacle lens to have an appropriate thickness, and easily secures processability and impact resistance, and is widely used.

### [Citation List]

### [Patent Literature]

[PTL 1] Japanese Patent Application Publication No. 2004-002820

### [Summary of Invention]

### [Technical Problem]

Isocyanates used as raw materials for plastic spectacle lenses generally have high reactivity, and readily become discolored when stored for a long time. In the fields such as optical materials, discolored isocyanates cannot be used.

One embodiment of the present disclosure relates to an isocyanate composition having high storage stability, a polymerizable composition, a resin, an optical material, and a spectacle lens.

### [Solution to Problem]

One embodiment of the present disclosure relates to an isocyanate composition including a compound represented by Formula (1): 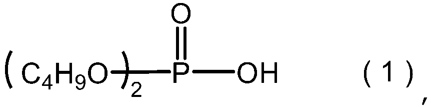 and
an isocyanate component.

One embodiment of the present disclosure relates to a polymerizable composition including
the above isocyanate composition, and
an active hydrogen compound component.

One embodiment of the present disclosure relates to
a resin which is a cured product of the above polymerizable composition.

One embodiment of the present disclosure relates to
an optical material containing the above resin.

One embodiment of the present disclosure relates to
a spectacle lens containing the above resin.

### [Advantageous Effects of Invention]

According to one embodiment of the present disclosure, it is possible to provide an isocyanate composition having high storage stability, a polymerizable composition, a resin, an optical material, and a spectacle lens.

### [Brief Description of Drawings]

### [Fig. 1]

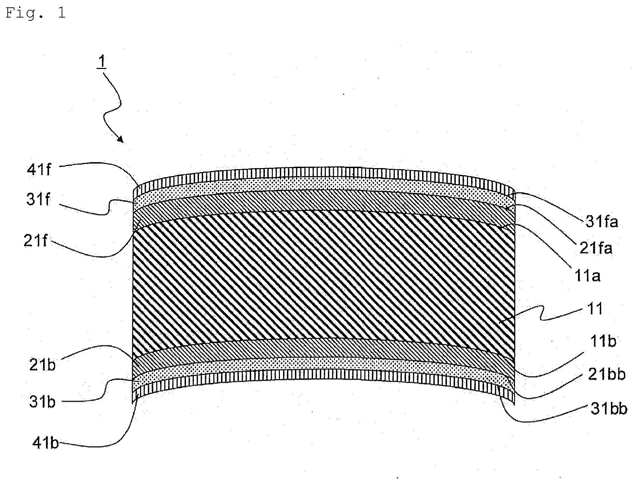

Fig. 1 is a schematic cross-sectional view of a spectacle lens 1 according to the present embodiment.

### [Description of Embodiments]

Hereinafter, embodiments according to the present disclosure will be described in detail with reference to the drawings as necessary, but the present disclosure is not limited thereto, and can be variously modified without departing from the scope and spirit thereof. Here, in the drawings, the same components are denoted with the same reference numerals and redundant descriptions are omitted. In addition, positional relationships such as above, below, left and right are based on the positional relationships shown in the drawings unless otherwise specified. In addition, dimensional ratios in the drawings are not limited to the illustrated ratios.

Here, in this specification, for example, the expression of a numerical value range of "1 to 100" includes both the lower limit value "1" and the upper limit value "100." In addition, the same applies to the expression of other numerical value ranges.

In addition, "a cured product of an isocyanate component and an active hydrogen compound component" does not mean that other components are excluded but means a cured product of a composition containing at least an isocyanate component and an active hydrogen compound component.

### [Isocyanate Composition]

An isocyanate composition according to the present embodiment includes a compound (hereinafter referred to as "DBP") represented by Formula (1): 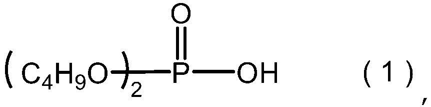 and
an isocyanate component.

According to the above configuration, an isocyanate composition having high storage stability is obtained.

The compound represented by Formula (1) can improve the storage stability of an isocyanate component by being mixed with the isocyanate component. The compound represented by Formula (1) exhibits excellent storage stability equivalent to that of 2,6-di-tert-butyl-p-cresol, which has conventionally been used as a storage stabilizer. Therefore, in one embodiment, it is possible to stabilize the isocyanate component without using 2,6-di-tert-butyl-p-cresol, which exhibits toxicity.

The content of the compound represented by Formula (1) in the isocyanate composition according to the present embodiment relative to a total amount of the isocyanate component is preferably 10 to 10,000 ppm by mass, more preferably 50 to 5,000 ppm by mass, and still more preferably 100 to 3,000 ppm by mass.

### (Isocyanate Component)

Examples of isocyanate components include polyisocyanate compounds having an aromatic ring, polyisocyanate compounds having an aliphatic ring, and linear or branched aliphatic polyisocyanate compounds.

Examples of polyisocyanate compounds having an aromatic ring include diisocyanatobenzene, 2,4-tolylene diisocyanate, 2,6-tolylene diisocyanate, ethyl phenylene diisocyanate, isopropyl phenylene diisocyanate, diethyl phenylene diisocyanate, diethyl phenylene diisocyanate, diisopropyl phenylene diisocyanate, trimethylbenzene triisocyanate, benzene triisocyanate, biphenyl diisocyanate, 4,4'-diphenylmethane diisocyanate, 4,4'-methylenebis(2-methylphenylisocyanate), bibenzyl-4,4'-diisocyanate, bis(isocyanatophenyl)ethylene, 1,3-bis(isocyanatomethyl)benzene, 1,4-bis(isocyanatomethyl)benzene, 1,3-bis(isocyanatoethyl)benzene, bis(isocyanatopropyl)benzene, α,α,α',α'-tetramethylxylylene diisocyanate, bis(isocyanatobutyl)benzene, bis(isocyanatomethyl)naphthalene, bis(isocyanatomethylphenyl)ether, 2-isocyanatophenyl-4-isocyanatophenyl sulfide, bis(4-isocyanatophenyl)sulfide, bis(4-isocyanatomethylphenyl)sulfide, bis(4-isocyanatophenyl)disulfide, bis(2-methyl-5-isocyanatophenyl)disulfide, bis(3-methyl-5-isocyanatophenyl)disulfide, bis(3-methyl-6-isocyanatophenyl)disulfide, bis(4-methyl-5-isocyanatophenyl)disulfide, bis(3-methyloxy-4-isocyanatophenyl)disulfide, and bis(4-methyloxy-3-isocyanatophenyl)disulfide.

Examples of polyisocyanate compounds having an aliphatic ring include 1,3-diisocyanatocyclohexane, 1,4-diisocyanatocyclohexane, isophorone diisocyanate, 1,3-bis(isocyanatomethyl)cyclohexane, 1,4-bis(isocyanatomethyl)cyclohexane, dicyclohexylmethane-4,4'-diisocyanate, dicyclohexylmethane-2,4'-diisocyanate, 2,5-bis(isocyanatomethyl)-bicyclo[2.2.1]heptane, 2,6-bis(isocyanatomethyl)-bicyclo[2.2.1]heptane, 2,5-diisocyanato-1,4-dithiane, 2,5-bis(isocyanatomethyl)-1,4-dithiane, 4,5-diisocyanato-1,3-dithiolane, 4,5-bis(isocyanatomethyl)-1,3-dithiolane, and 4,5-bis(isocyanatomethyl)-2-methyl-1,3-dithiolane.

Examples of linear or branched aliphatic polyisocyanate compounds include pentamethylene diisocyanate, hexamethylene diisocyanate, 2,2-dimethylpentane diisocyanate, 2,2,4-trimethylhexane diisocyanate, butene diisocyanate, 1,3-butadiene-1,4-diisocyanate, 2,4,4-trimethylhexamethylene diisocyanate, 1,6,11-undecane triisocyanate, 1,3,6-hexamethylenetriisocyanate, 1,8-diisocyanato-4-isocyanatomethyloctane, bis(isocyanatoethyl)carbonate, bis(isocyanatoethyl)ether, lysine diisocyanatomethyl ester, lysine triisocyanate, bis(isocyanatomethyl)sulfide, bis(isocyanatoethyl)sulfide, bis(isocyanatopropyl)sulfide, bis(isocyanatohexyl)sulfide, bis(isocyanatomethyl)sulfone, bis(isocyanatomethyl)disulfide, bis(isocyanatoethyl)disulfide, bis(isocyanatopropyl)disulfide, bis(isocyanatomethylthio)methane, bis(isocyanatoethylthio)methane, bis(isocyanatomethylthio)ethane, bis(isocyanatoethylthio)ethane, 1,5-diisocyanato-2-isocyanatomethyl-3-pentane, 1,2,3-tris(isocyanatomethylthio)propane, 1,2,3-tris(isocyanatoethylthio)propane, 3,5-dithia-1,2,6,7-heptanetetraisocyanate, 2,6-diisocyanatomethyl-3,5-dithia-1,7-heptane diisocyanate, 2,5-diisocyanatomethylthiophene, 4-isocyanatoethylthio-2,6-dithia-1,8-octanediisocyanate, 1,2-diisothiocyanatoethane, and 1,6-diisothiocyanatohexane.

These may be used alone or two or more thereof may be used.

The isocyanate component preferably includes at least one selected from the group consisting of bis(isocyanatomethyl)bicyclo[2.2.1]heptane, bis(isocyanatomethyl)cyclohexane, bis(isocyanatomethyl)benzene, tolylene diisocyanate, diphenylmethane diisocyanate, dicyclohexylmethane diisocyanate, hexamethylene diisocyanate, and pentamethylene diisocyanate (hereinafter referred to as a "preferred isocyanate compound").

Bis(isocyanatomethyl)bicyclo[2.2.1]heptane includes, for example, one or more selected from the group consisting of 2,5-bis(isocyanatomethyl)bicyclo[2.2.1]heptane and 2,6-bis(isocyanatomethyl)bicyclo[2.2.1]heptane, and is preferably a mixture of 2,5-bis(isocyanatomethyl)bicyclo[2.2.1]heptane and 2,6-bis(isocyanatomethyl)bicyclo[2.2.1]heptane.

Examples of bis(isocyanatomethyl)cyclohexane include 1,3-bis(isocyanatomethyl)cyclohexane and 1,4-bis(isocyanatomethyl)cyclohexane. Among these, 1,3-bis(isocyanatomethyl)cyclohexane is preferable.

Examples of bis(isocyanatomethyl)benzene include 1,3-bis(isocyanatomethyl)benzene and 1,4-bis(isocyanatomethyl)benzene. Among these, 1,3-bis(isocyanatomethyl)benzene is preferable.

Examples of tolylene diisocyanates include 2,4-tolylene diisocyanate and 2,6-tolylene diisocyanate. Among these, 2,4-tolylene diisocyanate is preferable.

Examples of diphenylmethane diisocyanates include 4,4'-diphenylmethane diisocyanate and 2,4'-diphenylmethane diisocyanate.

Examples of dicyclohexylmethane diisocyanates include dicyclohexylmethane-4,4'-diisocyanate.

In the isocyanate component, the content of the above "preferred isocyanate compound" is preferably 80 mass% or more, more preferably 90 mass% or more, and still more preferably 95 mass% or more and 100 mass% or less.

### (Phenol-based Antioxidant)

The isocyanate composition according to the present embodiment may contain a phenol-based antioxidant. When the compound represented by Formula (1) is used, excellent storage stability is exhibited even when used in combination with a phenol-based antioxidant.

Examples of phenol-based antioxidants include triethylene glycol bis[3-(3-tert-butyl-5-methyl-4-hydroxyphenyl)propionate], 1,6-hexanediol-bis[3-(3,5-di-tert-butyl-4-hydroxyphenyl)propionate], pentaerythritol tetrakis[3-(3,5-di-tert-butyl-4-hydroxyphenyl)propionate], octadecyl-3-(3,5-di-tert-butyl-4-hydroxyphenyl)propionate, 1,3,5-trimethyl-2,4,6-tris(3,5-di-tert-butyl-4-hydroxybenzyl)benzene, N,N-hexamethylenebis(3,5-di-tert-butyl-4-hydroxy-hydrocinnamamide), 3,5-di-tert-butyl-4-hydroxybenzylphosphonate-diethyl ester, tris(3,5-di-tert-butyl-4-hydroxybenzyl)isocyanurate, 3,9-bis{1,1-dimethyl-2-[β-(3-tert-butyl-4-hydroxy-5-methylphenyl)propionyloxy]ethyl}-2,4,8,10-tetraoxaspiro[5.5]undecane, 2,6-di-tert-butyl-p-cresol (hereinafter referred to as "BHT"), and phenol. Among these, 2,6-di-tert-butyl-p-cresol or phenol is preferable.

The content of the phenol-based antioxidant in the isocyanate composition according to the present embodiment relative to a total amount of the isocyanate component is preferably 10 to 10,000 ppm by mass, more preferably 30 to 1,000 ppm by mass, and still more preferably 50 to 500 ppm by mass.

### [Polymerizable Composition]

The polymerizable composition according to the present embodiment contains the above isocyanate composition and an active hydrogen compound component.

### (Active Hydrogen Compound Component)

Examples of active hydrogen compound components include polythiol components, polyol components, and polyamine components.

### (Polythiol Component)

Examples of polythiol components include an ester compound of a polyol compound and a mercapto group-containing carboxylic acid compound, a linear or branched aliphatic polythiol compound, a polythiol compound having an aliphatic ring, and a polythiol compound having an aromatic ring.

In the ester compound of a polyol compound and a mercapto group-containing carboxylic acid compound, examples of polyol compounds include compounds containing two or more hydroxyl groups in the molecule. Here, examples of polyol compounds include ethylene glycol, diethylene glycol, propanediol, propanetriol, butanediol, trimethylolpropane, bis(2-hydroxyethyl)disulfide, pentaerythritol, and dipentaerythritol.

Examples of mercapto group-containing carboxylic acid compounds include thioglycolic acid, mercaptopropionic acid, a thiolactic acid compound, and thiosalicylic acid.

Examples of ester compounds of a polyol compound and a mercapto group-containing carboxylic acid compound include ethylene glycol bis(2-mercaptoacetate), ethylene glycol bis(3-mercaptopropionate), diethylene glycol bis(2-mercaptoacetate), diethylene glycol bis(3-mercaptopropionate), 1,4-butanediol bis(2-mercaptoacetate), 1,4-butanediol bis(3-mercaptopropionate), trimethylolpropane tris(2-mercaptoacetate), trimethylolpropane tris(3-mercaptopropionate), pentaerythritol tetrakis(2-mercaptoacetate), pentaerythritol tetrakis(3-mercaptopropionate), dipentaerythritol hexakis(2-mercaptoacetate), and dipentaerythritol hexakis(3-mercaptopropionate).

Examples of linear or branched aliphatic polythiol compounds include 1,2-ethanedithiol, 1,1-propanedithiol, 1,2-propanedithiol, 1,3-propanedithiol, 2,2-propanedithiol, 1,6-hexanedithiol, 1,2,3-propanetrithiol, 2,2-dimethylpropane-1,3-dithiol, 3,4-dimethyloxybutane-1,2-dithiol, 2,3-dimercapto-1-propanol, 1,2-dimercaptopropyl methyl ether, 2,3-dimercaptopropyl methyl ether, dimercaptoethyl ether, 2-(2-mercaptoethylthio)propane-1,3-dithiol, 2,2-bis(mercaptomethyl)-1,3-propanedithiol, bis(mercaptomethylthio)methane, tris(mercaptomethylthio)methane, bis(2-mercaptoethylthio)methane, 1,2-bis(mercaptomethylthio)ethane, 1,2-bis(2-mercaptoethylthio)ethane, 1,3-bis(mercaptomethylthio)propane, 1,3-bis(2-mercaptoethylthio)propane, 1,1,2,2-tetrakis(mercaptoethylthio)ethane, 1,1,3,3-tetrakis(mercaptoethylthio)propane, 3-mercaptomethyl-1,5-dimercapto-2,4-dithiapentane, tetrakis(mercaptoethylthio)propane, bis(2-mercaptoethyl)ether, bis(2-mercaptoethyl)sulfide, bis(2-mercaptoethyl)disulfide, 1,2-bis(2-mercaptoethylthio)-3-mercaptopropane, 4,7-bis(mercaptomethyl)-3,6,9-trithiaundecane-1,11-dithiol, 4,8-bis(mercaptomethyl)-3,6,9-trithiaundecane-1,11-dithiol, and 5,7-bis(mercaptomethyl)-3,6,9-trithiaundecane-1,11-dithiol.

Examples of polythiol compounds having an aliphatic ring include 1,1-cyclohexanedithiol, 1,2-cyclohexanedithiol, methylcyclohexanedithiol, bis(mercaptomethyl)cyclohexane, 2-(2,2-bis(mercaptomethylthio)ethyl)-1,3-dithiethane, 2,5-bis(mercaptomethyl)-1,4-dithiane, and 4,8-bis(mercaptomethyl)-1,3-dithiane.

Examples of polythiol compounds having an aromatic ring include 1,3-dimercaptobenzene, 1,4-dimercaptobenzene, 1,3-bis(mercaptomethyl)benzene, 1,4-bis(mercaptomethyl)benzene, 1,3-bis(mercaptoethyl)benzene, 1,4-bis(mercaptoethyl)benzene, 1,3,5-trimercaptobenzene, 1,3,5-tris(mercaptomethyl)benzene, 1,3,5-tris(mercaptoethyl)benzene, 4,4'-dimercaptobiphenyl, 4,4'-dimercaptobibenzyl, 2,5-toluenedithiol, 1,5-naphthalenedithiol, 2,6- naphthalenedithiol, 2,7-naphthalenedithiol, 2,4-dimethylbenzene-1,3-dithiol, 4,5-dimethylbenzene-1,3-dithiol, 9,10-anthracenedimethanethiol, 1,3-di(p-methyloxyphenyl)propane-2,2-dithiol, 1,3-diphenylpropane-2,2-dithiol, phenylmethane-1,1-dithiol, and 2,4-di(p-mercaptophenyl)pentane.

These may be used alone or two or more thereof may be used.

### (Polyol Component)

Examples of polyol components include ethylene glycol, diethylene glycol, propanediol, propanetriol, butanediol, trimethylolpropane, bis(2-hydroxyethyl)disulfide, pentaerythritol, and dipentaerythritol.

### (Polyamine Component)

Examples of polyamine components include polymethylenediamine, polyetherdiamine, diethylenetriamine, iminobispropylamine, bishexamethylenetriamine, diethylenetriamine, tetraethylenepentamine, pentaethylenehexaamine, pentaethylenehexaamine, dimethylaminopropylamine, aminoethylethanolamine, methyliminobispropylamine, methanediamine, N-aminomethylbiperazine, 1,3-diaminocyclohexane, isophoronediamine, meta-xylenediamine, tetrachloroparaxylylenediamine, metaphenylenediamine, 4,4'-methylenedianiline, diaminodiphenylsulfone, benzidine, diaminodiphenyl ether, 4,4'-thiodianiline, 4,4'-bis(o-toluidine)dianisidine, o-phenylenediamine, 2,4-toluenediamine, 2,5-toluenediamine, methylenebis(o-chloroaniline), diaminiditolylsulfone, bis(3,4-diaminophenyl)sulfone, 2,6-diaminopyridine, 4-chloro-o-phenylenediamine, 4-methoxy-6-methyl-m-phenylenediamine, m-aminobenzylamine, N,N,N',N'-tetramethyl-1,3-butanediamine, N,N,N',N'-tetramethyl-p-phenylenediamine, tetramethylguanidine, 2-dimethylamino-2-hydroxypropane, pyrazine, 2,4,6-tris(dimethylaminomethylol)phenol, N-methylpiperazine, N-β(aminoethyl)γ-aminopropyltrimethoxysilane, N-β(aminoethyl)γ-aminopropylmethyldimethoxysilane, and γ-aminopropylmethyldimethoxysilane.

The active hydrogen compound component preferably includes at least one selected from the group consisting of toluenediamine, pentaerythritol tetrakis(mercaptoacetate), pentaerythritol tetrakis(mercaptopropionate), trimethylolpropane tris(mercaptoacetate), trimethylolpropane tris(mercaptopropionate), bis(mercaptoethylthio)mercaptopropane, bis(mercaptomethyl)-3,6,9-trithiaundecanedithiol, dimercaptoethyl sulfide, bis(mercaptomethyl)dithiane, dimercaptoethyl ether and diethylene glycol.

Examples of toluenediamines include 2,4-toluenediamine and 2,5-toluenediamine.

Examples of pentaerythritol tetrakis(mercaptoacetate) include pentaerythritol tetrakis(2-mercaptoacetate).

Examples of pentaerythritol tetrakis(mercaptopropionate) include pentaerythritol tetrakis(3-mercaptopropionate).

Examples of trimethylolpropane tris(mercaptoacetate) include trimethylolpropane tris(2-mercaptoacetate).

Examples of trimethylolpropane tris(mercaptopropionate) include trimethylolpropane tris(3-mercaptopropionate).

Examples of bis(mercaptoethylthio)mercaptopropane include 1,2-bis(2-mercaptoethylthio)-3-mercaptopropane.

Examples of bis(mercaptomethyl)-3,6,9-trithiaundecanedithiol include 4,7-bis(mercaptomethyl)-3,6,9-trithiaundecane-1,11-dithiol, 4,8-bis(mercaptomethyl)-3,6,9-trithiaundecane-1,11-dithiol, 5,7-bis(mercaptomethyl)-3,6,9-trithiaundecane-1,11-dithiol. Bis(mercaptomethyl)-3,6,9-trithiaundecanedithiol is preferably a mixture of 4,7-bis(mercaptomethyl)-3,6,9-trithiaundecane-1,11-dithiol, 4,8-bis(mercaptomethyl)-3,6,9-trithiaundecane-1,11-dithiol, and 5,7-bis(mercaptomethyl)-3,6,9-trithiaundecane-1,11-dithiol.

The active hydrogen compound component is preferably a polythiol component.

The polythiol component
includes preferably at least one selected from the group consisting of 2,5-bis(mercaptomethyl)-1,4-dithiane, pentaerythritol tetrakis(2-mercaptoacetate), pentaerythritol tetrakis(3-mercaptopropionate), 4-mercaptomethyl-1,8-dimercapto-3,6-dithiaoctane, 4,7-bis(mercaptomethyl)-3,6,9-trithiaundecane-1,11-dithiol, 4,8-bis(mercaptomethyl)-3,6,9-trithiaundecane-1,11-dithiol, 5,7-bis(mercaptomethyl)-3,6,9-trithiaundecane-1,11-dithiol, trimethylolpropane tris(2-mercaptoacetate), trimethylolpropane tris(3-mercaptopropionate), butanediol bis(2-mercaptoacetate), butanediol bis(3-mercaptopropionate), dipentaerythritol hexakis(2-mercaptoacetate), and dipentaerythritol hexakis(3-mercaptopropionate);
more preferably at least one selected from the group consisting of 4,7-bis(mercaptomethyl)-3,6,9-trithiaundecane-1,11-dithiol, 4,8-bis(mercaptomethyl)-3,6,9-trithiaundecane-1,11-dithiol, 5,7-bis(mercaptomethyl)-3,6,9-trithiaundecane-1,11-dithiol, 4-mercaptomethyl-1,8-dimercapto-3,6-dithiaoctane, pentaerythritol tetrakis(3-mercaptopropionate), 2,5-bis(mercaptomethyl)-1,4-dithiane, and pentaerythritol tetrakis(2-mercaptoacetate);
still more preferably at least one selected from the group consisting of 4,7-bis(mercaptomethyl)-3,6,9-trithiaundecane-1,11-dithiol, 4,8-bis(mercaptomethyl)-3,6,9-trithiaundecane-1,11-dithiol, and 5,7-bis(mercaptomethyl)-3,6,9-trithiaundecane-1,11-dithiol; and
yet more preferably a mixture of 4,7-bis(mercaptomethyl)-3,6,9-trithiaundecane-1,11-dithiol, 4,8-bis(mercaptomethyl)-3,6,9-trithiaundecane-1,11-dithiol, and 5,7-bis(mercaptomethyl)-3,6,9-trithiaundecane-1,11-dithiol.

The amount of the above preferable polythiol component in the polythiol component is preferably 50 mass% or more, more preferably 70 mass% or more, still more preferably 90 mass% or more, and yet more preferably 95 mass% or more, and 100 mass% or less.

The equivalent ratio of mercapto groups of the polythiol component and isocyanato groups of the polyisocyanate component (mercapto group/isocyanato group) is preferably 40/60 or more, more preferably 43/57 or more, still more preferably 45/55 or more, and preferably 60/40 or less, more preferably 55/45 or less, and still more preferably 53/47 or less.

A total content of the polythiol component and the polyisocyanate component in the polymerizable composition is preferably 80 mass% or more, more preferably 90 mass% or more, still more preferably 95 mass% or more, and 100 mass% or less.

The polymerizable composition according to the present embodiment may contain other additives such as a UV absorbing agent, a coloring agent, an antioxidant, a coloring inhibitor, and a fluorescent brightening agent. These may be used alone or two or more thereof may be used.

### [Resin]

The resin according to the present embodiment is a cured product of the above polymerizable composition. The cured product is obtained by polymerizing the polymerizable composition.

Polymerization conditions can be appropriately set according to the polymerizable composition. The polymerization start temperature is preferably 0°C or higher, more preferably 10°C or higher, and preferably 50°C or lower, and more preferably 40°C or lower. Preferably, the temperature is raised from the polymerization start temperature, and curing by heating is then performed. For example, the maximum heating temperature is usually 110°C or higher and 130°C or lower.

The resin is used, for example, as an optical material. The optical material includes the above resin. The resin is preferably used as a spectacle lens among optical materials.

### [Spectacle Lens]

The spectacle lens according to the present embodiment includes, for example, a lens substrate containing the resin according to the present embodiment. The spectacle lens according to the present embodiment may include at least one layer selected from the group consisting of a hard coat layer, a foundation layer, and an antireflection layer.

Fig. 1 is a schematic cross-sectional view of a spectacle lens 1 according to the present embodiment. The spectacle lens 1 according to the present embodiment includes a lens substrate 11, a hard coat layer 21f provided on the side of an object-side surface 11a of the lens substrate 11, a functional layer 31f provided on the side of an object-side surface 21fa of the hard coat layer 21f, and an antifouling layer 41f provided on the side of an object-side surface 31fa of the functional layer 31f.

In addition, when the lens substrate 11 is a finished lens, the spectacle lens 1 according to the present embodiment further includes a hard coat layer 21b provided on the side of an eyeball-side surface 11b of the lens substrate 11, a functional layer 31b provided on the side of an eyeball-side surface 21bb of the hard coat layer 21b, and a water-repellent layer 41b provided on the side of an eyeball-side surface 31bb of the functional layer 31b.

Here, although not shown, a foundation layer may be provided between the lens substrate 11 and the hard coat layer 21f or between the lens substrate 11 and the hard coat layer 21b.

### <Lens Substrate>

The lens substrate may contain the resin according to the present embodiment.

The surface shape of the lens substrate is not particularly limited, and may be flat, convex, concave or the like.

The lens substrate may be used for any application such as a single focal lens, a multifocal lens, and a progressive power lens. For example, as one example, for a progressive power lens, usually, a near portion region (near portion) and a progressive portion region (intermediate region) are included in the above lower region, and a distance portion region (distance portion) is included in an upper region.

As the lens substrate, a colorless lens substrate is usually used, but a lens substrate that is colored in a range in which the transparency is not impaired can also be used.

The lens substrate is preferably of a meniscus type. A "meniscus type" lens substrate means a lens substrate having a curved surface formed on both sides. When the meniscus type lens substrate contains the above Compound 1, it is possible to reduce astigmatism.

The optical center thickness of the lens substrate is not particularly limited, and is preferably 0.5 mm or more and 10.0 mm or less, more preferably 0.5 mm or more and 5.0 mm or less, still more preferably 0.5 mm or more and 3.0 mm or less, and yet more preferably 0.5 mm or more and 2.0 mm or less.

The diameter of the lens substrate is not particularly limited, and is usually about 50 to 100 mm.

The refractive index ne of the lens substrate is preferably 1.52 or more, more preferably 1.53 or more, still more preferably 1.55 or more, yet more preferably 1.58 or more, and even more preferably 1.60 or more.

In order to enhance an effect of improving the Abbe number according to the inclusion of Compound 1, the refractive index ne of the lens substrate is preferably 1.70 or more, and more preferably 1.74 or more.

Here, although the upper limit of the refractive index ne of the lens substrate is not particularly limited, it may be, for example 1.80 or less.

### [Method of Producing Lens Substrate]

Although not particularly limited, the lens substrate is obtained by, for example, a production method including
a step of curing the above polymerizable composition and
a step of annealing a cured resin.

The polymerization is preferably a cast polymerization method. For example, the lens substrate can be obtained by injecting a polymerizable composition into a mold in which a glass or metal mold and a tape or a gasket are combined and performing polymerization.

Polymerization conditions can be appropriately set according to the polymerizable composition. The polymerization start temperature is preferably 0°C or higher, more preferably 10°C or higher, and preferably 50°C or lower, and more preferably 40°C or lower. Preferably, the temperature is raised from the polymerization start temperature, and curing by heating is then performed. For example, the maximum heating temperature is usually 110°C or higher and 130°C or lower.

After polymerization is completed, the lens substrate may be released from the mold and subjected to an annealing treatment. The temperature in the annealing treatment is preferably 100 to 150°C.

### <Hard Coat Layer>

The hard coat layer is, for example, a cured film formed of a curable composition containing an inorganic oxide and a silicon compound. The curable composition preferably further contains a polyfunctional epoxy compound.

Examples of inorganic oxides include silicon oxide, aluminum oxide, titanium oxide, zirconium oxide, tungsten oxide, zinc oxide, tin oxide, beryllium oxide, antimony oxide, and composite oxides formed of two or more of these inorganic oxides. These may be used alone or two or more thereof may be used in combination. Among these inorganic oxides, silicon oxide is preferable. Here, colloidal silica may be used as the inorganic oxide.

The content of the inorganic oxide in the solid content of the curable composition is preferably 20 mass% or more and 80 mass% or less, more preferably 25 mass% or more and 70 mass% or less, and still more preferably 25 mass% or more and 50 mass% or less.

The silicon compound is, for example, a silicon compound having a hydrolyzable group such as an alkoxy group. The silicon compound is preferably a silane coupling agent containing an organic group and a hydrolyzable group bonded to silicon atoms. The organic group bonded to silicon atoms is preferably an organic group having a functional group, for example, an epoxy group such as a glycidoxy group, a vinyl group, a methacryloxy group, an acryloxy group, a mercapto group, an amino group, and a phenyl group, and more preferably an organic group having an epoxy group. Here, the silicon compound may have an alkyl group bonded to silicon.

Examples of commercial products of the above silane coupling agents include KBM-303, KBM-402, KBM-403, KBE-402, KBE-403, KBM-1403, KBM-502, KBM-503, KBE-502, KBE-503, KBM-5103, KBM-602, KBM-603, KBM-903, KBE-903, KBE-9103, KBM-573, KBM-575, KBM-9659, KBE-585, KBM-802, KBM-803, KBE-846, and KBE-9007 (product name, commercially available from Shin-Etsu Chemical Co., Ltd.).

The content of the silicon compound in the solid content of the curable composition is preferably 20 mass% or more and 90 mass% or less, more preferably 30 mass% or more and 75 mass% or less, and still more preferably 50 mass% or more and 75 mass% or less.

The polyfunctional epoxy compound is a polyfunctional epoxy compound containing two or more epoxy groups in one molecule, and more preferably a polyfunctional epoxy compound containing two or three epoxy groups in one molecule. Examples of commercial products of polyfunctional epoxy compounds include "Denacol" series EX-201, EX-211, EX-212, EX-252, EX-313, EX-314, EX-321, EX-411, EX-421, EX-512, EX-521, EX-611, EX-612, EX-614, and EX-614B (product name, commercially available from Nagase ChemteX Corporation).

The content of the polyfunctional epoxy compound in the solid content of the curable composition is preferably 0 mass% or more and 50 mass% or less, more preferably 10 mass% or more and 40 mass% or less, and still more preferably 15 mass% or more and 30 mass% or less.

The above curable composition can be prepared by mixing optional components such as an organic solvent, a leveling agent, and a curing catalyst as necessary in addition to the components described above.

The above hard coat layer can be formed by applying a curable composition to the substrate and performing a curing treatment (thermal curing, photocuring, etc.). As a method of applying a curable composition, commonly used methods such as a dipping method, a spin coating method, and a spray method can be applied. The curing treatment is usually performed by performing heating on a curable composition containing a polyfunctional epoxy compound. For example, the heat curing treatment can be performed by disposing the lens coated with the above curable composition under an environment of an atmospheric temperature of 50 to 150°C for about 30 minutes to 3 hours.

### <Foundation Layer>

For example, the above foundation layer can be formed from an aqueous resin composition containing at least one resin particle selected from the group consisting of a polyurethane resin, an acrylic resin, and an epoxy resin.

As the above aqueous resin composition, a commercially available aqueous polyurethane can be used without change or one that is diluted with an aqueous solvent as necessary can be used. Examples of commercially available aqueous polyurethanes include "Evafanol" series (product name, commercially available from Nicca Chemical Co., Ltd.), "SuperFlex" series (product name, commercially available from DKS Co., Ltd.), "Adeka Bontighter" series (product name, commercially available from ADEKA Corporation), "Olester" series (product name, commercially available from Mitsui Chemicals Inc), "Bondic" series and "Hydran" series (product name, commercially available from Dainippon Ink and Chemicals, Inc.), "Impranil" series (product name, commercially available from Bayer AG), "Soflanate" series (product name, commercially available from Japan Soflan Co., Ltd.), "Poiz" series (product name, commercially available from Kao Corporation), "Sanprene" series (product name, commercially available from Sanyo Chemical Industries, Ltd.), "Aizerax" series (product name, commercially available from Hodogaya Chemical Co., Ltd.), and "NeoRez" series (product name, commercially available from AstraZeneca).

The foundation layer can be formed, for example, by applying the above aqueous resin composition to the surface of the substrate and drying it.

### <Functional Layer>

Examples of the above functional layers include an antireflection layer, an ultraviolet light absorbing layer, an infrared light absorbing layer, a photochromic layer, an antistatic layer, and an anti-fogging layer. These functional layers may be used alone or two or more thereof may be used in combination. For these functional layers, known techniques related to spectacle lenses can be applied. Among these, it is preferable to have an antireflection layer.

### (Antireflection Layer)

For example, the antireflection layer has low refractive index layers and high refractive index layers that are alternately arranged. The number of layers that the antireflection layer has is preferably 4 to 11 and more preferably 5 to 8.

The refractive index of the low refractive index layer is preferably 1.35 to 1.80 and more preferably 1.45 to 1.50 at a wavelength of 500 to 550 nm. The low refractive index layer is formed of an inorganic oxide and preferably formed of silicon oxide.

The refractive index of the high refractive index layer is preferably 1.90 to 2.60 and more preferably 2.00 to 2.40 at a wavelength of 500 to 550 nm. The high refractive index layer is formed of, for example, an inorganic oxide. The inorganic oxide used for the high refractive index layer is preferably at least one selected from the group consisting of zirconium oxide, tantalum oxide, yttrium oxide, titanium oxide, niobium oxide and aluminum oxide, and more preferably at least one selected from the group consisting of zirconium oxide and tantalum oxide.

For the antireflection layer, low refractive index layers and high refractive index layers can be alternately laminated by a vacuum deposition method to form an antireflection layer.

### <Water-repellent Layer>

The water-repellent layer is formed using a water-repellent material composition to be described below. The water-repellent layer may be formed on the hard coat layer or formed on the functional layer, but is preferably formed on the antireflection layer. Moreover, the water-repellent layer is preferably positioned on the outermost surface.

As described above, this specification discloses the following embodiments.
<1> An isocyanate composition including:
   a compound represented by Formula (1): 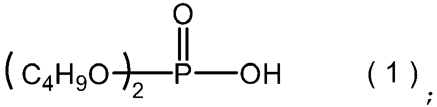 and
   an isocyanate component.
<2> The isocyanate composition according to <1> or <2>,
   wherein the amount of the compound represented by Formula (1) added is 10 to 10,000 ppm by mass relative to the amount of the isocyanate component.
<3> The isocyanate composition according to <1> or <2>,
   wherein the isocyanate component includes at least one selected from the group consisting of bis(isocyanatomethyl)bicyclo[2.2.1]heptane, bis(isocyanatomethyl)cyclohexane, bis(isocyanatomethyl)benzene, tolylene diisocyanate, diphenylmethane diisocyanate, dicyclohexylmethane diisocyanate, hexamethylene diisocyanate, and pentamethylene diisocyanate.
<4> The isocyanate composition according to any of <1> to <3>, containing a phenol-based antioxidant.
<5> The isocyanate composition according to <4>,
   wherein the phenol-based antioxidant contains 2,6-di-tert-butyl-p-cresol or phenol.
<6> The isocyanate composition according to <4> or <5>,
   wherein the amount of the phenol-based antioxidant added is 10 to 10,000 ppm by mass relative to the amount of the isocyanate component.
<7> A polymerizable composition including the isocyanate composition according to any of <1> to <6>; and
   an active hydrogen compound component.
<8> The polymerizable composition according to <7>,
   wherein the active hydrogen compound component includes at least one selected from the group consisting of toluenediamine, pentaerythritol tetrakis(mercaptoacetate), pentaerythritol tetrakis(mercaptopropionate), trimethylolpropane tris(mercaptoacetate), trimethylolpropane tris(mercaptopropionate), bis(mercaptoethylthio)mercaptopropane, bis(mercaptomethyl)-3,6,9-trithiaundecanedithiol, dimercaptoethyl sulfide, bis(mercaptomethyl)dithiane, dimercaptoethyl ether and diethylene glycol.
<9> The polymerizable composition according to <8>,
   wherein the bis(mercaptomethyl)-3,6,9-trithiaundecanedithiol is a mixture of 4,7-bis(mercaptomethyl)-3,6,9-trithiaundecane-1,11-dithiol, 4,8-bis(mercaptomethyl)-3,6,9-trithiaundecane-1,11-dithiol and 5,7-bis(mercaptomethyl)-3,6,9-trithiaundecane-1,11-dithiol.
<10> A resin which is a cured product of the polymerizable composition according to <8> or <9>.
<11> An optical material including the resin according to <10>.
<12> A spectacle lens including the resin according to <10> or <11>.

### [Examples]

Hereinafter, the present embodiment will be described in more detail with reference to examples and comparative examples. Here, the present invention is not limited to the following examples.

### [Hazen Color Number (APHA)]

The Hazen color number (APHA) of the polymerizable composition was measured by the method of JIS K 0071-1:1998.

### <Example 1>

To a mixture of 2,5-bis(isocyanatomethyl)-bicyclo[2.2.1]heptane and 2,6-bis(isocyanatomethyl)-bicyclo[2.2.1]heptane (hereinafter referred to as "I-1") as an isocyanate component, 1,000 ppm by mass of dibutyl phosphate (compound represented by Formula (1), hereinafter referred to as "DBP") relative to the amount of the isocyanate component was added to obtain an isocyanate composition. The APHA of the obtained isocyanate composition was evaluated and shown as 0 day in Table 1. The samples were stored under a temperature condition of 40°C and the APHAs after 7 days, 14 days, and 21 days were measured and shown in Table 1.

### <Example 2>

An isocyanate composition was obtained in the same manner as in Example 1 except that 50 ppm by mass of 2,6-di-tert-butyl-p-cresol (hereinafter referred to as "BHT") was additionally added to the isocyanate component. The isocyanate composition was subjected to APHA evaluation in the same manner as in Example 1.

### <Example 3>

An isocyanate composition was obtained in the same manner as in Example 1 except that 100 ppm by mass of BHT was additionally added to the isocyanate component. The isocyanate composition was subjected to APHA evaluation in the same manner as in Example 1.

### <Example 4>

An isocyanate composition was obtained in the same manner as in Example 1 except that 50 ppm by mass of phenol (hereinafter referred to as "PhOH") was additionally added to the isocyanate component. The isocyanate composition was subjected to APHA evaluation in the same manner as in Example 1.

### <Example 5>

An isocyanate composition was obtained in the same manner as in Example 1 except that 100 ppm by mass of PhOH was additionally added to the isocyanate component. The isocyanate composition was subjected to APHA evaluation in the same manner as in Example 1.

### <Comparative Example 1>

An isocyanate composition was obtained in the same manner as in Example 1 except that, in place of DBP, 50 ppm by mass of BHT was added to the isocyanate component. The isocyanate composition was subjected to APHA evaluation in the same manner as in Example 1.

### <Comparative Example 2>

An isocyanate composition was obtained in the same manner as in Example 1 except that, in place of DBP, 1,000 ppm by mass of butoxyethyl acid phosphate "JP-506H" (product name, commercially available from Johoku Chemical Co., Ltd.) was added to the isocyanate component. The isocyanate composition was subjected to APHA evaluation in the same manner as in Example 1.

### <Comparative Example 3>

An isocyanate composition was obtained in the same manner as in Comparative Example 2 except that 50 ppm by mass of BHT was additionally added to the isocyanate component. The isocyanate composition was subjected to APHA evaluation in the same manner as in Example 1.

### <Comparative Example 4>

An isocyanate composition was obtained in the same manner as in Comparative Example 2 except that 100 ppm by mass of BHT was additionally added to the isocyanate component. The isocyanate composition was subjected to APHA evaluation in the same manner as in Example 1.

### <Comparative Example 5>

An isocyanate composition was obtained in the same manner as in Comparative Example 2 except that 50 ppm by mass of PhOH was additionally added to the isocyanate component. The isocyanate composition was subjected to APHA evaluation in the same manner as in Example 1.

### <Comparative Example 6>

An isocyanate composition was obtained in the same manner as in Comparative Example 2 except that 100 ppm by mass of PhOH was additionally added to the isocyanate component. The isocyanate composition was subjected to APHA evaluation in the same manner as in Example 1.

**[Table 1]**

| Table 1 (1/2) | | | | | | | |
|---|---|---|---|---|---|---|---|
| | | | Example 1 | Example 2 | Example 3 | Example 4 | Example 5 |
| Isocyanate composition | Isocyanate component | | I-1 | I-1 | I-1 | I-1 | I-1 |
| | Compound (1) | Type | DBP | DBP | DBP | DBP | DBP |
| | | Amount (ppm by mass) | 1000 | 1000 | 1000 | 1000 | 1000 |
| | Compound (2) | Type | - | BHT | BHT | PhOH | PhOH |
| | | Amount (ppm by mass) | - | 50 | 100 | 50 | 100 |
| APHA | 0day | | 8 | 8 | 8 | 8 | 8 |
| | 7days | | 8 | 8 | 8 | 8 | 8 |
| | 14days | | 9 | 9 | 9 | 9 | 9 |
| | 21days | | 9 | 10 | 10 | 9 | 10 |

| Table 1 (2/2) | | | | | | | | |
|---|---|---|---|---|---|---|---|---|
| | | | Comparative Example 1 | Comparative Example 2 | Comparative Example 3 | Comparative Example 4 | Comparative Example 5 | Comparative Example 6 |
| Isocyanate composition | Isocyanate component | | I-1 | I-1 | I-1 | I-1 | I-1 | I-1 |
| | Compound (1) | Type | - | JP506H | JP506H | JP506H | JP506H | JP506H |
| | | Amount (ppm by mass) | - | 1000 | 1000 | 1000 | 1000 | 1000 |
| | Compound (2) | Type | BHT | - | BHT | BHT | PhOH | PhOH |
| | | Amount (ppm by mass) | 50 | - | 50 | 100 | 50 | 100 |
| APHA | 0day | | 8 | 8 | 8 | 8 | 8 | 8 |
| | 7days | | 8 | 10 | 10 | 10 | 10 | 10 |
| | 14days | | 8 | 12 | 12 | 12 | 12 | 12 |
| | 21days | | 9 | 13 | 13 | 13 | 13 | 13 |

The various abbreviations in the table are as follows.
I-1: a mixture of 2,5-bis(isocyanatomethyl)-bicyclo[2.2.1]heptane and 2,6-bis(isocyanatomethyl)-bicyclo[2.2.1]heptane
DBP: dibutyl phosphate (compound represented by Formula (1))
BHT: 2,6-di-tert-butyl-p-cresol
JP-506H: butoxyethyl acid phosphate
Amount (ppm by mass): amount added relative to the amount of the isocyanate component (unit: ppm by mass)

### <Example 6>

0.06 parts by mass of dimethyltin dichloride as a catalyst, 0.15 parts by mass of butoxyethyl acid phosphate "JP-506H" (product name, commercially available from Johoku Chemical Co., Ltd.) as a mold release agent, and 0.55 parts by mass of a UV absorbing agent "SEESORB 701"(product name, commercially available from Shipro Kasei Kaisha, Ltd.) were added to 50.28 parts by mass of the isocyanate composition of Example 1, and the mixture was stirred and mixed. Then, 25.50 parts by mass of pentaerythritol tetrakis(3-mercaptopropionate), and 24.22 parts by mass of 1,2-bis(2-mercaptoethylthio)-3-mercaptopropane were additionally added, and the mixture was stirred and mixed under a reduced pressure of 10 mmHg for 30 minutes to prepare a curable composition. Next, this curable composition was injected into a lens molding mold composed of a glass mold and a resin gasket prepared in advance (0.00D, the wall thickness was set to 1.6 mm) and polymerized in an electric furnace at 20°C to 120°C for 24 hours. After the polymerization was completed, the gasket and the mold were removed, a heat treatment was then performed at 120°C for 2 hours to obtain a lens substrate.

## Claims

1. An isocyanate composition comprising:
a compound represented by Formula (1): 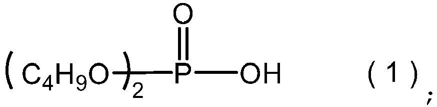 and
an isocyanate component.

2. The isocyanate composition according to claim 1,
wherein the amount of the compound represented by Formula (1) added is 10 to 10,000 ppm by mass relative to the amount of the isocyanate component.

3. The isocyanate composition according to claim 1,
wherein the isocyanate component includes at least one selected from the group consisting of bis(isocyanatomethyl)bicyclo[2.2.1]heptane, bis(isocyanatomethyl)cyclohexane, bis(isocyanatomethyl)benzene, tolylene diisocyanate, diphenylmethane diisocyanate, dicyclohexylmethane diisocyanate, hexamethylene diisocyanate, and pentamethylene diisocyanate.

4. The isocyanate composition according to claim 1, comprising a phenol-based antioxidant.

5. The isocyanate composition according to claim 4,
wherein the phenol-based antioxidant contains 2,6-di-tert-butyl-p-cresol or phenol.

6. The isocyanate composition according to claim 4,
wherein the amount of the phenol-based antioxidant added is 10 to 10,000 ppm by mass relative to the amount of the isocyanate component.

7. A polymerizable composition comprising:
the isocyanate composition according to any one of claims 1 to 6; and
an active hydrogen compound component.

8. The polymerizable composition according to claim 7,
wherein the active hydrogen compound component includes at least one selected from the group consisting of toluenediamine, pentaerythritol tetrakis(mercaptoacetate), pentaerythritol tetrakis(mercaptopropionate), trimethylolpropane tris(mercaptoacetate), trimethylolpropane tris(mercaptopropionate), bis(mercaptoethylthio)mercaptopropane, bis(mercaptomethyl)-3,6,9-trithiaundecanedithiol, dimercaptoethyl sulfide, bis(mercaptomethyl)dithiane, dimercaptoethyl ether and diethylene glycol.

9. The polymerizable composition according to claim 8,
wherein the bis(mercaptomethyl)-3,6,9-trithiaundecanedithiol is a mixture of 4,7-bis(mercaptomethyl)-3,6,9-trithiaundecane-1,11-dithiol, 4,8-bis(mercaptomethyl)-3,6,9-trithiaundecane-1,11-dithiol and 5,7-bis(mercaptomethyl)-3,6,9-trithiaundecane-1,11-dithiol.

10. A resin which is a cured product of the polymerizable composition according to claim 8.

11. An optical material comprising the resin according to claim 10.

12. A spectacle lens comprising the resin according to claim 10.
